Europäisches Patentamt

(19) European Patent Office

Office européen des brevets

(11) Publication number: **0 024 628**
**B1**

(12) **EUROPEAN PATENT SPECIFICATION**

(45) Date of publication of patent specification: 03.04.85

(21) Application number: 80104769.7

(22) Date of filing: 12.08.80

(51) Int. Cl.[4]: **C 07 D 301/32,**
**C 07 D 301/10,**
**C 07 D 303/04, C 07 D 317/38**

(54) **Preparation of ethylene carbonate.**

(30) Priority: 17.08.79 US 67580
24.10.79 US 87841

(43) Date of publication of application:
11.03.81 Bulletin 81/10

(45) Publication of the grant of the patent:
03.04.85 Bulletin 85/14

(84) Designated Contracting States:
BE DE FR GB IT NL

(56) References cited:
GB-A-1 204 181
GB-A-1 213 484
US-A-2 773 070
US-A-3 228 968

CHEMICAL ABSTRACTS, vol. 93, no. 2, July 14,
1980, page 379, abstract 13903s. Columbus,
Ohio, USA M.C. ANNESINI et al. "Solubility of
carbon dioxide in ethylene carbonate and
ethylene oxide"

(73) Proprietor: THE DOW CHEMICAL COMPANY
Dow Center 2030 Abbott Road Post Office Box
1967
Midland Michigan 48640 (US)

(72) Inventor: Tsang, Albert Cheuk-kei
1718 Boulevard De Province
Baton Rouge Louisiana (US)
Inventor: Ainsworth, Oliver Clarence
3552 Bahin Court
Baton Rouge Louisiana (US)
Inventor: Raines, Dale A.
55 Harlan
Lakewood Colorado (US)

(74) Representative: Casalonga, Axel
BUREAU D.A. CASALONGA OFFICE JOSSE &
PETIT Baaderstrasse 12-14
D-8000 München 5 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The process of manufacturing ethylene oxide (EO) by oxidation of ethylene with oxygen in the vapor phase produces a product stream which is very dilute in ethylene oxide, i.e., one to two percent. The ethylene oxide is normally recovered by absorbing in water and employs a large counter-current tower for the purpose. Carbon dioxide, which is a by-product, is also absorbed from the product stream, but not as completely as the EO. The water containing the absorbed EO is then passed to a stripping column where the EO is recovered and the water recycled to the absorbing tower.

The absorption process is energy intensive, i.e., the absorbing water must be maintained at about 35°C in order to accommodate the heat load from the reactor gases and efficiently absorb the EO. Further, the temperature required to desorb the EO from the aqueous solution is about 95°C. At this temperature some of the water evaporates which is a further waste of energy.

It would be highly desirable if an absorption system could be devised which would be less energy intensive and employ smaller apparatus and equipment.

GB—A—1 204 181 discloses a liquid phase preparation process of propylene oxide according to which the gas is contacted with a liquid exhactant comprising propylene glycol or an ester or ether thereof.

GB—A—1 213 484 relates to the preparation of ethylene oxide by scrubbing with a non aqueous liquid absorbant such as ethane, methane, ethylene, halogenated hydrocarbon.

The gas stream from the reactor which contains the ethylene oxide also contains carbon dioxide, oxygen, water, and unreacted ethylene. Ethylene carbonate has a greater affinity for EO and absorbs it efficiently at a higher temperature than does water, thus making it unnecessary to cool the absorbing medium to as low a temperature to accommodate the heat of reaction present in the effluent gases. Thus, the operating temperature for absorbing EO in ethylene carbonate is about 35°C to 65°C, contrasted with water at 30°C to 40°C. Ethylene carbonate is a better absorbent than water for EO and $CO_2$, i.e., a given volume of absorbent will hold more of the absorbed gases at a given temperature when ethylene carbonate is employed in place of water. Also, $CO_2$ is more efficiently absorbed with respect to EO in ethylene carbonate compared to water, i.e., the ratio $CO_2$/EO absorbed is greater in ethylene carbonate than in water. Thus, $CO_2$ in the recycle gas to the EO reactor will be less. Also the specific heat of ethylene carbonate is about one-third that of water, so the heat applied in the stripping operation to recover the EO will be considerably less.

Since ethylene carbonate and water are miscible in all proportions, water is also absorbed by the ethylene carbonate, but it is readily stripped therefrom by the application of heat and by employing an inert stripping gas.

The composition of the effluent gases from the ethylene oxide process will generally fall within the following ranges of components given as mole percent:

$CO_2$ . . . . . . . . . . . . . . . . . . . . .0.2—12

$C_2H_4$ . . . . . . . . . . . . . . . . . . . .0.2—98

$O_2$. . . . . . . . . . . . . . . . . . . . . .0.2—7

$H_2O$ . . . . . . . . . . . . . . . . . . . . .0.5—3

EO . . . . . . . . . . . . . . . . . . . . . .0.4—5

$N_2$. . . . . . . . . . . . . . . . . . . . . . 0—98

The above ranges can vary widely since the invention is applicable to ethylene oxide processes employing air as the source of oxygen as well as oxygen-enriched air or pure oxygen, and also processes employing high ratios of ethylene to oxygen, wherein ethylene makes up a substantial amount of the feed and effluent gases.

Although ethylene, oxygen, and nitrogen are not absorbed to any great extent by ethylene carbonate, the relative amounts of these gases in the effluent affects the actual amount absorbed. Thus, with high ratios of ethylene or in processes using pure oxygen, more of these gases will be absorbed.

The pressure of the gas in the absorbing column is substantially that at which the EO reactor is run and is determined thereby.

The liquid/vapor (mole) ratios for the absorbing system are operable from about 0.2 to about 1.0 and are preferably from about 0.35 to about 0.70. For the desorption or stripping system the L/V ratios operate at from about 10—150 with the preferred range being from about 50 to about 125.

## Preparation of Ethylene Carbonate

The preparation of alkylene carbonates by reacting an alkylene oxide and carbon dioxide is well-known. The general conditions for the reaction are the use of temperatures in the approximate range of 100°C—250°C. Superatmospheric pressures of about 10—300 atmospheres are employed. A reaction temperature of about 160°C—200°C and a pressure of 50—150 atmospheres are usually preferred. The reactants are used in about equal molar proportions with the carbon dioxide normally in slight excess.

Known catalysts for the reaction include inorganic bases such as sodium hydroxide and sodium carbonate and organic nitrogen bases such as tertiary amines, quaternary ammonium bases, and salts of these nitrogen bases such as their carbonates and halides. For example, aliphatic tertiary amines such as trimethylamine, aromatic tertiary amines such as pyridine and quinoline, quaternary ammonium hydroxides such as tetraethyl ammonium hydroxide, trimethyl benzyl ammonium hydroxide, dialkyl piperidinium hydroxide, and the carbonates, bicarbonates, and halides of such hydroxides are all known to catalyze the reaction. Catalyst concentrations of 0.1—5 percent based on the weight of alkylene oxide are conventional.

Other catalysts disclosed in the patent literature are anion-exchange resins containing quaternary ammonium chloride groups (U.S. Patent 2,773,070). Catalysts known to the art generally are effective for the purpose and they provide fairly high conversions of the reactants and generally good yields of the desired cyclic carbonates. These yields usually are about 70—90 percent of the theoretical.

The manufacture of alkylene carbonates is effectively accomplished in conjunction with the preparation of alkylene oxides. Thus, for example, ethylene carbonate production is advantageously located near an ethylene oxide plant.

The ethylene oxide obtained from the direct oxidation of ethylene is usually not pure enough to be employed as a feed to an ethylene carbonate plant without prior purification. It has now been discovered that when ethylene oxide is recovered from the effluent of such a plant by absorbing it in ethylene carbonate, the ethylene oxide together with the carbon dioxide absorbed can be employed without further purification as feed to a process for making ethylene carbonate. Thus, a simple stripping and drying of these gases from the ethylene carbonate absorbent provides reactants of acceptable purity for this process.

Accordingly, the present invention is a process for preparing ethylene carbonate characterized by (1) oxidizing ethylene with oxygen in the presence of a silver catalyst to produce ethylene oxide, (2) contacting the effluent gases from said reaction with ethylene carbonate to absorb ethylene oxide and carbon dioxide, (3) stripping said ethylene oxide and carbon dioxide from the ethylene carbonate absorbent by contacting with an inert gas, which may be carbon dioxide (4) reacting said ethylene oxide and carbon dioxide over an anion-exchange resin to form ethylene carbonate, and (5) recovering said ethylene carbonate.

By the invention referred to immediately above, an improved integrated process is obtained by absorbing the reactor effluent in ethylene carbonate, desorbing (stripping) the $CO_2$ and EO from the ethylene carbonate absorbent and, thereafter, without further purification except for a drying step, employing these gases as feed to an ethylene carbonate manufacturing process.

A molar excess of $CO_2$ over the stoichiometric requirements is preferred.

The effluent of an ethylene oxide reactor is contacted with ethylene carbonate, whereby ethylene oxide, water, and carbon dioxide are absorbed. The absorbed gases are desorbed by employing heat and stripping with an inert gas (preferably $CO_2$), drying and feeding the desorbed $CO_2$ and EO, together with sufficient additional $CO_2$ to provide an excess over the stoichiometric amount, to a reactor containing a catalyst for the reaction of EO and $CO_2$ to produce ethylene carbonate. A preferred catalyst is a strong anion-exchange resin containing trimethylbenzyl ammonium chloride groups. Other strong anion-exchange resins containing quaternary ammonium groups are also useful. The product ethylene carbonate is separated from any unconverted reactants (which are recycled to the ethylene carbonate reactor) and subsequently distilled to remove any higher boiling impurities, e.g., polycarbonates.

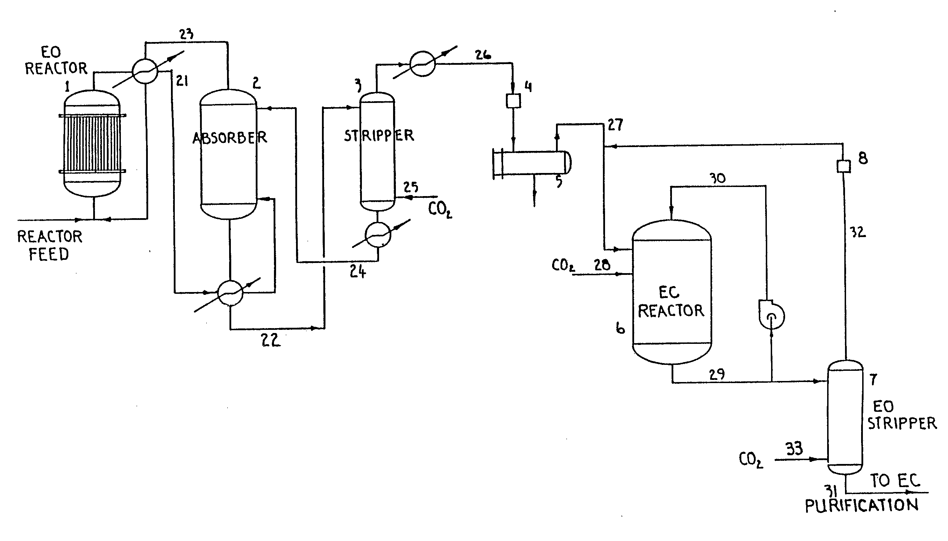

The absorption of the ethylene oxide by ethylene carbonate and its removal therefrom, and when desired, the preparation of ethylene carbonate by reacting EO with $CO_2$ in the presence of a strong anion-exchange resin, as part of the integrated process, is described by reference to the drawing as follows:

The effluent of the EO reactor 1 containing 0.05—5 volume percent ethylene oxide is passed via conduit 21 to an absorption (absorber) column 2 containing ethylene carbonate (maintained at a temperature of 35°C—65°C and a pressure of $11,38 \times 10^5$—$25,18 \times 10^5$ Pa (165—365 Psia) wherein EO and $CO_2$ are absorbed; the ethylene which is not absorbed is returned via conduit 23 to the EO reactor. The EC absorbent containing dissolved EO and $CO_2$ is then sent via conduit 22 to a desorption (stripper) column 3 where it is heated to and maintained at a temperature of 90°C—150°C and a pressure of $0,130 \times 10^5$—$1,03 \times 10^5$ Pa (2—15 Psia) and contacted with an inert stripping gas ($CO_2$ or $N_2$) introduced via line 25 at a temperature of about 110°C. The stripped gases (EO and $CO_2$) are removed through conduit 26 and the ethylene carbonate absorbent is recycled via conduit 24 to absorber 2. The stripped gases are compressed to a pressure of $6,9 \times 10^5$—$34,5 \times 10^5$ Pa (100—500 Psia) by compressor 4 and sent through condenser 5 (maintained at a temperature of 0°C—25°C) to condense out water prior to being fed via conduit 27 to the ethylene carbonate reactor 6 which contains an anion-exchange resin catalyst. The pressure in reactor 6 is maintained at about $6,9 \times 10^5$—$34,5 \times 10^5$ Pa (100—500 Psia) but somewhat lower than that of the incoming feed gases. Additional $CO_2$ is fed to the reactor (if needed to provide an adequate molar excess) via line 28. The ethylene carbonate is removed via conduit 29, excess $CO_2$ is recycled to the ethylene carbonate reactor via conduit 30 and any dissolved unreacted EO is removed from the ethylene carbonate in EO stripper 7 to which $CO_2$ is fed via conduit 33 as a stripping gas. The stripping gas is also used as a source of make-up $CO_2$ to the reactor 6 and is returned thereto via conduit 32 and passed through compressor 8 where the gases are raised to a pressure sufficient to overcome the pressure

3

in reactor 6 to which they are fed as reactants, while the product ethylene carbonate is sent to a purification step via conduit 31.

The following examples illustrate the invention.

Examples 1—3 — Absorption

An effluent gas stream from an ethylene oxide reactor, containing ethylene oxide, carbon dioxide, small amounts of ethylene, oxygen and water with the remainder being nitrogen, was passed through a column containing ethylene carbonate as the absorbent. Some of the carbon dioxide, but little or no ethylene, nitrogen, and oxygen were absorbed. The ethylene oxide and water were substantially completely absorbed. Table 1 shows the conditions of temperature pressure and the liquid/vapor ratio (mole) together with the percentage of EO and $CO_2$ removed from the thus treated gas stream.

Table I

| Example No. | Temp (°C) | Press Pa Psia | L/V (mole) | Vol. % in Effluent EO | $CO_2$ | Percent Absorbed EO | $CO_2$ |
|---|---|---|---|---|---|---|---|
| 1 | 50 | $15,52 \times 10^5$ (225) | 0.45 | 1.03 | 9.26 | 99.6 | 5.3 |
| 2 | 50 | $15,52 \times 10^5$ (225) | 0.37 | 0.99 | 9.08 | 97.9 | 4.5 |
| 3 | 50 | $15,87 \times 10^5$ 259 | 0.60 | 0.66 | 8.94 | 98.5 | 2.8 |

Examples 4 and 5 — Stripping

Ethylene carbonate, which has been employed to absorb EO and $CO_2$ from the effluent of an ethylene oxide reactor, was passed to a stripping column where it was heated and contacted with an inert stripping gas (nitrogen). Table II shows the conditions, amounts of absorbed gases, and the percentages of EO and water removed.

Table II

| Example No. | Wt. % Absorbate EO | $H_2O$ | Temp (°C) | Press, Pa Psia | L/V (mole) | Wt. % Removed EO | $H_2O$ |
|---|---|---|---|---|---|---|---|
| 4 | 1.0 | 1.0 | 97 | $0,83 \times 10^5$ 12 | 61 | 95 | 24.0 |
| 5 | 0.83 | 1.1 | 95 | $0,65 \times 10^5$ 10 | 108 | 93 | 25.5 |

Substantially all of the $CO_2$ absorbed by the ethylene carbonate was removed by the stripping process. Only a few parts per million remained.

Example 6

A synthetic gas mixture, representative of a stream stripped from an ethylene carbonate absorber of the effluent of an EO plant wherein air is used as the oxygen source, which contained about 1 mole percent EO, 9 mole percent $CO_2$ and 2 mole percent water vapor, was compressed and then sent through a condenser to remove the water. Thereafter the gas stream was passed over a catalyst in a reaction vessel which consisted of a packed bed of an anion-exchange resin containing trimethylbenzyl ammonium chloride groups. Additional $CO_2$ was added to the reactor to produce a reaction mixture containing 8.3 mole percent EO, 91.7 mole percent $CO_2$, and 260 ppm (wt.) acetaldehyde. The resin bed had a volume of 95 ml and the process parameters were:

flow rate = (14.16 1/hr (0.5 scf/hr)

temperature = 105°C—106°C

pressure = $24,15 \times 10^5$ Pa (350 Psig)

At substantially 100 percent selectivity the yield was 87 percent ethylene carbonate.

4

# 0 024 628

## Examples 7—11

In the same equipment and in the manner of Example 6, but employing different ratios of reactants, reaction parameters and catalysts, other experiments were conducted which are shown in the following table. Mole percent of EO alone is given, the balance consisting of $CO_2$.

| Ex. | EO, Mole % | Acetaldehyde (ppm) | Exch* Resin | Press, Pa (Psig) | Temp (°C) | Flow, (1/hr) (SCF/hr) | % Yield, EC |
|---|---|---|---|---|---|---|---|
| 7 | 14.0 | 260 | A | 15,18 220 | 95 | (43.0) 1.52 | 45 |
| 8 | 13.0 | 130 | A | 15,18 220 | 100 | (28.3) 1.00 | 69 |
| 9 | 1.8 | — | B | 15,18 220 | 105 | (102) 3.60 | 79 |
| 10 | 1.8 | — | D | 15,18 220 | 115 | (50.2) 1.77 | 89 |
| 11 | 1.6 | — | C | 15,18 220 | 115 | (50.2) 1.77 | 92 |

*A, B and C are anion-exchange resins containing trimethylbenzyl ammonium groups. D is a silane fluid which contains N-(2-aminoethyl)-3-aminipropyl trimethoxy-silane. This was coated on alumina pellets and treated with bromopropane to quaternize the amine groups.

## Claims

1. A process for preparing ethylene carbonate characterized by (1) oxidizing ethylene with oxygen in the presence of a silver catalyst to produce ethylene oxide, (2) contacting the effluent gases from said reaction with ethylene carbonate to absorb ethylene oxide and carbon dioxide, (3) stripping said ethylene oxide and carbon dioxide from the ethylene carbonate absorbent by contacting with an inert gas, which may be $CO_2$, (4) reacting said ethylene oxide and sufficient carbon dioxide, if necessary added from another source, over an anion exchange resin to form ethylene carbonate, and (5) recovering said ethylene carbonate.

2. The process of Claim 1 further characterized in that additional carbon dioxide is supplied to the reactor to provide a stoichiometric excess of carbon dioxide with respect to ethylene oxide therein.

3. The process of Claim 2 further characterized in that unreacted carbon dioxide is recovered and recycled to the ethylene carbonate reactor.

4. The process of Claim 1 further characterized in that stripping step (3) is achieved by heating and employing carbon dioxide as the inert gas.

5. The process of Claim 1 further characterized in that the ethylene oxide and carbon dioxide stripped from the absorbent are dried prior to passing into the ethylene carbonate reactor.

6. The process of Claim 1 further characterized in that the anion-exchange resin contains trimethylbenzyl ammonium halide groups.

## Revendications

1. Procédé de préparation du carbonate d'éthylène caractérisé en ce qu'il consiste à (1) oxyder l'éthylène avec de l'oxygène en présence d'un catalyseur à l'argent pour produire de l'oxyde d'éthylène, (2) mettre en contact les gaz effluents de ladite réaction avec du carbonate d'éthylène pour absorber l'oxyde d'éthylène et le dioxyde de carbone, (3) réextraire ledit oxyde d'éthylène et ledit dioxyde de carbone de l'absorbant carbonate d'éthylène par une mise en contact avec un gaz inerte qui peut être du dioxyde de carbone, (4) faire réagir ledit oxyde d'éthylène et une quantité suffisante de dioxyde de carbone, introduite éventuellement en provenance d'une autre source, sur une résine échangeuse d'anions pour former le carbonate d'éthylène et (5) récupérer ledit carbonate d'éthylène.

2. Procédé selon la revendication 1, caractérisé en outre en ce que le dioxyde de carbone supplémentaire est apporté au réacteur pour fournir un excès stoechiométrique de dioxyde de carbone par rapport à l'oxyde d'éthylène contenu dans celui ci.

3. Procédé selon la revendication 2, caractérisé en outre en ce que le dioxyde de carbone non réagi est récupéré et recyclé vers le réacteur à carbonate d'éthylène.

4. Procédé selon la revendication 1, caractérisé en outre en ce que l'étape de rectification (3) est réalisée en chauffant et en employant le dioxyde de carbone comme gaz inerte.

5. Procédé selon la revendication 1, caractérisé en outre en ce que l'oxyde d'éthylène et le dioxyde de carbone réextraits de l'absorbant sont séchés avant de passer dans le réacteur à carbonate d'éthylène.

6. Procédé selon la revendication 1, caractérisé en outre en ce que la résine échangeuse d'anions contient des groupes halogénure de triméthylbenzylammonium.

## Patentansprüche

1. Ein Verfahren zur Herstellung von Ethylencarbonat, gekennzeichnet durch:

(1) Oxidation von Ethylen mit Sauerstoff in Gegenwart eines Silberkatalysators zwecks Bildung von Ethylenoxid,

(2) Inberührungbringen der bei der genannten Reaktion freiwerdenden Gase mit Ethylencarbonat zwecks Absorbierens von Ethylenoxid und Kohlendioxid,

(3) Abstreifen des genannten Ethylenoxids und Kohlendioxids von dem Ethylencarbonatabsorptionsmittel durch Inberührungbringen mit einem inerten Gas, das $CO_2$ sein kann,

(4) Reagierenlassen des genannten Ethylenoxids und einer hinreichenden Menge Kohlendioxid, das ggf. aus einer anderen Quelle zugesetzt wird, über einem Anionenaustauscherharz zwecks Bildung von Ethylencarbonat, und

(5) Gewinnung des genannten Ethylencarbonats.

2. Verfahren gemäß Anspruch 1, ferner dadurch gekennziechnet, daß zusätzliches Kohlendioxid in den Reaktor eingespeist wird, um einen stöchiometrischen Überschuß gegenüber dem darin enthaltenen Ethylenoxid zu erreichen.

3. Verfahren gemäß Anspruch 2, dadurch gekennzeichnet, daß unreagiertes Kohlendioxid abgezogen und in den Ethylencarbonatreaktor rückgeführt wird.

4. Verfahren gemäß Anspruch 1, dadurch gekennzeichnet, daß der Abstreifschritt (3) durch Aufheizen und Einsatz von Kohlendioxidgas als inertes Gas erreicht, wird.

5. Verfahren gemäß Anspruch 1, dadurch gekennzeichnet, daß das vom Absorptionsmittel abgestreifte Ethylenoxid und das Kohlendioxid getrocknet werden bevor sie dem Ethylencarbonatreaktor zugeführt werden.

6. Verfahren gemäß Anspruch 1, dadurch gekennzeichnet, daß das Anionenaustauscherharz Trimethylbenzylammoniumhalogenidgruppen enthält.